# EUROPEAN PATENT APPLICATION

(11) **EP 3 103 426 A1**
(43) Date of publication of application: **14.12.2016**
(21) Application number: 15743359.0
(22) Date of filing: 28.01.2015
(51) Int. Cl.: A61H 3/00, A61F 2/62, B25J 11/00

(54) **WEARABLE ACTION ASSISTANCE DEVICE**

(30) Priority: 30.01.2014 JP 2014015798
(71) Applicant: University of Tsukuba, Tsukuba-shi, Ibaraki 305-8577 (JP); Cyberdyne Inc., Ibaraki 305-0818 (JP)
(72) Inventor: SANKAI, Yoshiyuki, Tsukuba-shi Ibaraki 305-8577 (JP); TAKAMA, Masahiro, Tsukuba-shi Ibaraki 305-0818 (JP); TANAKA, Hiroshi, Tsukuba-shi Ibaraki 305-0818 (JP)
(74) Representative: Zacco Sweden AB
(86) International application number: PCT/JP2015/052379
(87) International publication number: WO 2015/115490

(57) **Abstract**

A wearable action-assistance device is provided that can be fitted to and worn by various wearers who have different body types and sizes. A wearable action-assistance device (10) has a hip frame (11) that is to be worn on the hips of a wearer, a lower limb frame (12) that is to be worn on lower limbs of the wearer, and drive portions (13R, 13L, 14R, 14L) that are provided on the lower limb frame (12) in correspondence with joints of the wearer. The lower limb frame (12) has variable shape portions (41L, 41R, 42L, 42R, 43L, 43R, 44L, 44R, 45L and 45R) that can cause a plurality of places including portions in the vicinity of the drive portions (13R, 13L, 14R, 14L) to bend to the left and right to adjust flexion angles thereof. By being able to bend a plurality of places on the lower limb frame (12) to the left and right, the shape of the lower limb frame (12) can be adjusted so as to fit the leg shapes of even knock-kneed or bowlegged wearers.

## Description

### Technical Field

The present invention relates to a wearable action-assistance device that assists actions of the wearer.

### Background Art

Known wearable action-assistance devices include devices that assist a standing-up action and a walking action of people who are challenged in their lower limb motor function who have difficulty in walking because of a decrease in the muscular strength of their skeletal muscles, or people who have difficulty in walking independently, such as patients undergoing walking motion rehabilitation (Patent Documents 1 and 2).

Such kinds of wearable action-assistance devices are configured to detect changes in biopotentials due to muscular activity of the wearer and measured at the skin surface, and by driving an actuator based on the detection result, the device can voluntarily control an actuator in accordance with the intention of the wearer, autonomously control an actuator in conformity with pre-programmed leg trajectories, or control an actuator in accordance with the motion of joints of the wearer. Hence, such kinds of wearable action-assistance devices are extremely useful for nursing care of physically disabled people and elderly people and the like, and also for rehabilitation of injured or sick people.

### Document List

Patent Documents
Patent Document 1: Japanese Patent No. 4997614
Patent Document 2: Japanese Laid-Open Patent Publication No. 2005-95561

### Summary of Invention

### Technical Problem

To accurately transmit an assistive force produced by a wearable action-assistance device to a wearer, it is important that the wearable action-assistance device is fitted closely with the body of the wearer.

Therefore, conventional wearable action-assistance devices are equipped with a plurality of belts for fixing a frame portion of the device to the hips, thighs, lower legs and the like of the wearer, and the wearable action-assistance device is fitted closely with the body of the wearer by adjusting the degree of tightening of the respective belts.

However, since there are wearers of various body types and sizes, there are not a few wearers for whom such devices do not fit by simply adjusting the degree of tightness of the belts, and such wearers may complain of a sense of discomfort when using the devices.

A problem to be solved by the present invention is to provide a wearable action-assistance device that can be fitted to and worn by various wearers of different body types and sizes.

### Solution to Problem

To solve the above described problem, the present invention has the following means.

A wearable action-assistance device of the present invention includes:
a hip frame that is to be worn on hips of a wearer;
a lower limb frame that is to be worn on a lower limb of the wearer;
a plurality of drive portions provided on the lower limb frame in correspondence with joints of the wearer; and
a control portion that controls the drive portions based on a signal that is caused by an action of the wearer,
wherein the lower limb frame has a variable shape portion which can bend a portion other than the drive portions to left and right to change a shape thereof.

According to the above described configuration, a lower limb frame can be bent to the left and right at places other than places at which drive portions are provided in correspondence with joints such as the knee and foot joints of a wearer, and thus the shape of the lower limb frame can be changed.

Therefore, in the wearable action-assistance device of the present invention, the shape of the lower limb frame can be appropriately changed to match the body type of the wearer, and can be fitted to and worn by various wearers of different body types and sizes. According to this configuration, the shapes of the respective portions of the lower limb frame can be changed to fit the leg shapes of, for example, knock-kneed wearers and bowlegged wearers also.

In the wearable action-assistance device of the present invention, it is preferable that, the hip frame is a member which is open in front and can accept the hips of the wearer and surround the hips from a rear part to both side parts on the left and right thereof, and has a hip width adjustment mechanism for adjusting a degree of opening in a left-right direction.

By adjusting the degree of opening of the hip frame, the wearable action-assistance device configured as described above can be fitted to and worn by various wearers of different body types and sizes.

In the wearable action-assistance device of the present invention, it is preferable that the lower limb frame has:
a right lower limb frame that is to be worn on a right lower limb of the wearer, and
a left lower limb frame that is to be worn on a left lower limb of the wearer;
the right lower limb frame has:
   a right thigh frame which is positioned on a right side of a right thigh of the wearer, and
   a right lower leg frame which is positioned on a right side of a right lower leg of the wearer;
   the left lower limb frame has:
a left thigh frame which is positioned on a left side of a left thigh of the wearer, and
a left lower leg frame which is positioned on a left side of a left lower leg of the wearer; and
the variable shape portion is provided at an intermediate portion in a vertical direction of each of the right thigh frame, the left thigh frame, the right lower leg frame and the left lower leg frame, and also in an upper vicinity of each of the drive portions.

The wearable action-assistance device configured as described above has variable shape portions at a total of 10 places, which consist of five places on the right lower limb frame and five places on the left lower limb frame. By appropriately changing the shape of any one or more variable shape portion among these variable shape portions to match the body type of the wearer, the wearable action-assistance device can be fitted to and worn by various wearers of different body types and sizes.

In the wearable action-assistance device of the present invention, it is preferable that, the lower limb frame has:
a right lower limb frame that is to be worn on a right lower limb of the wearer;
the right lower limb frame has:
   a right thigh frame that is positioned on a right side of a right thigh of the wearer, and
   a right lower leg frame that is positioned on a right side of a right lower leg of the wearer; and
the variable shape portion is provided at an intermediate portion in a vertical direction of each of the right thigh frame and the right lower leg frame, and also in an upper vicinity of each of the drive portions.

The wearable action-assistance device configured as described above has variable shape portions at five places on the right lower limb frame. By appropriately changing the shape of any one or more variable shape portion among these variable shape portions to match the body type of the wearer, the wearable action-assistance device can be fitted to and worn by various wearers of different body types and sizes.

In the wearable action-assistance device of the present invention, it is preferable that, the lower limb frame has:
a left lower limb frame that is to be worn on a left lower limb of the wearer;
the left lower limb frame has:
   a left thigh frame that is positioned on a left side of a left thigh of the wearer, and
   a left lower leg frame that is positioned on a left side of a left lower leg of the wearer; and
   the variable shape portion is provided at an intermediate portion in a vertical direction of each of the left thigh frame and the left lower leg frame, and also in an upper vicinity of each of the drive portions.

The wearable action-assistance device configured as described above has variable shape portions at five places on the left lower limb frame. By appropriately changing the shape of any one or more variable shape portion among these variable shape portions to match the body type of the wearer, the wearable action-assistance device can be fitted to and worn by various wearers of different body types and sizes.

In the wearable action-assistance device of the present invention, it is preferable that, the right thigh frame and the left thigh frame are connected to the hip frame via an elastic body, the right thigh frame and the left thigh frame being turnable around respective long axes thereof.

The wearable action-assistance device configured as described above can assist actions of a wearer while allowing turning motions (inward turning and outward turning) of the hip joints of the wearer.

Preferably, the wearable action-assistance device of the present invention has a turn limiting mechanism that limits a turning angle of the right thigh frame and the left thigh frame with respect to the hip frame to a predetermined angle or less.

The wearable action-assistance device configured as described above can prevent hip joints of a wearer from turning excessively.

Preferably, the wearable action-assistance device of the present invention has an assistive force exerting member that is attached to the lower limb frame to cause a force of the drive portion to act on the wearer from the front or rear.

The wearable action-assistance device configured as described above can efficiently cause an assistive force to act on the lower limbs of a wearer from the front or rear thereof, and thereby assist actions of the wearer.

Preferably, in the wearable action-assistance device of the present invention, the assistive force exerting member is configured to be attachable in a manner such that an orientation thereof with respect to the wearer can be appropriately changed to a forward-facing orientation or a rearward-facing orientation.

In the wearable action-assistance device configured as described above, since the assistive force exerting member can be attached so as to have an orientation that is efficient for causing an assistive force to act, the assistive force can be caused to act on the lower limbs of the wearer and assist actions of the wearer with favorable efficiency.

In the wearable action-assistance device of the present invention, it is preferable that a height of an attachment position of the assistive force exerting member with respect to the lower limb frame is adjustable.

In the wearable action-assistance device configured as described above, since the height of an attachment position of the assistive force exerting member can be adjusted in accordance with the body type and size of the wearer, an assistive force can be caused to act with greater efficiency on the lower limbs of the wearer to thereby assist actions of the wearer.

In the wearable action-assistance device of the present invention, it is preferable that an angle of the assistive force exerting member with respect to the lower limb frame is adjustable.

In the wearable action-assistance device configured as described above, since an angle of the assistive force exerting member can be adjusted in accordance with the body type and size of the wearer, an assistive force can be caused to act with greater efficiency on the lower limbs of the wearer to thereby assist actions of the wearer.

In the wearable action-assistance device of the present invention, it is preferable that, the assistive force exerting member is supported by the lower limb frame in a manner in which the assistive force exerting member is retractable to a position at which the assistive force exerting member does not interfere with the wearer.

In the wearable action-assistance device configured as described above, retracting the assistive force exerting member to a position at which the assistive force exerting member does not interfere with the wearer allows the wearable action-assistance device to be easily put onto and taken off from the wearer.

In the wearable action-assistance device of the present invention, it is preferable that, the assistive force exerting member is a member having a mounting face which is curved in an arc shape that can be arranged so as to fit to a lower limb of the wearer.

In the wearable action-assistance device configured as described above, since a mounting face that is curved in an arc shape of the assistive force exerting member fits on a lower limb of the wearer, an assistive force can be caused to act with greater efficiency on the lower limbs of the wearer to thereby assist actions of the wearer.

### Effects of Invention

A wearable action-assistance device of the present invention can be fitted to and worn by various wearers of different body types and sizes.

### Brief Description of Drawings

[Fig. 1] Fig. 1 is an overall perspective view illustrating an example of an embodiment of a wearable action-assistance device of the present invention.
[Fig. 2] Fig. 2 is a front view of the wearable action-assistance device.
[Fig. 3] Fig. 3 is a back view of the wearable action-assistance device.
[Fig. 4] Fig. 4 is a right side view of the wearable action-assistance device.
[Fig. 5] Fig. 5 is a perspective view illustrating a portion constituted by a hip frame and a back face unit of the wearable action-assistance device.
[Fig. 6] Fig. 6 is a side view of a principal portion of a lower limb frame of the wearable action-assistance device.
[Fig. 7] Fig. 7 is a perspective view illustrating a portion of the wearable action-assistance device at which a thigh cuff and a thigh cuff support mechanism are provided.
[Fig. 8] Fig. 8 is a perspective view of a principal portion that illustrates an attachment structure of the thigh cuff support mechanism.
[Fig. 9] Fig. 9 is a back view of a principal portion which illustrates that an angle of the thigh cuff support mechanism is adjustable.
[Fig. 10] Fig. 10 is a perspective view of a principal portion that illustrates an attachment structure of the thigh cuff support mechanism.
[Fig. 11] Fig. 11 is a perspective view illustrating a portion of the wearable action-assistance device at which a lower-leg cuff and a lower-leg cuff support mechanism are provided.
[Fig. 12] Fig. 12 is a perspective view of a principal portion that illustrates an attachment structure of the lower-leg cuff support mechanism.
[Fig. 13] Fig. 13 is a back view of a principal portion which illustrates that a height position of the lower-leg cuff support mechanism is adjustable.
[Fig. 14] Fig. 14 is a perspective view of the lower-leg cuff support mechanism.
[Fig. 15] Fig. 15 is a perspective view illustrating a state at a time of wearing the wearable action-assistance device.
[Fig. 16] Fig. 16 is a perspective view of an operation unit.
[Fig. 17] Fig. 17 is a front view of the operation unit.
[Fig. 18] Fig. 18 illustrates a settings screen that is displayed on a display of the operation unit.
[Fig. 19] Figs. 19A to 19C illustrate operation screens during assistance which are displayed on the operation unit.

### Description of the Embodiments

Hereinafter, preferred embodiments of the present invention will be described.

### A. Configuration of Wearable Action-Assistance Device

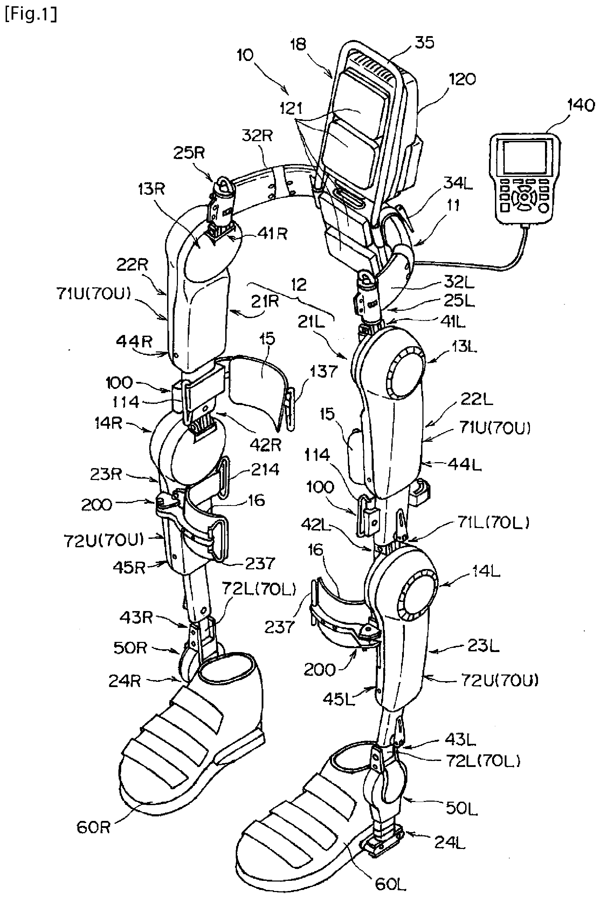

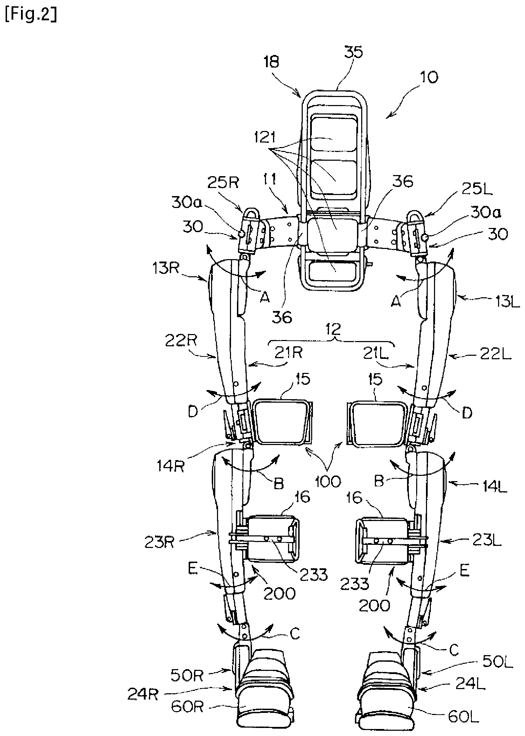

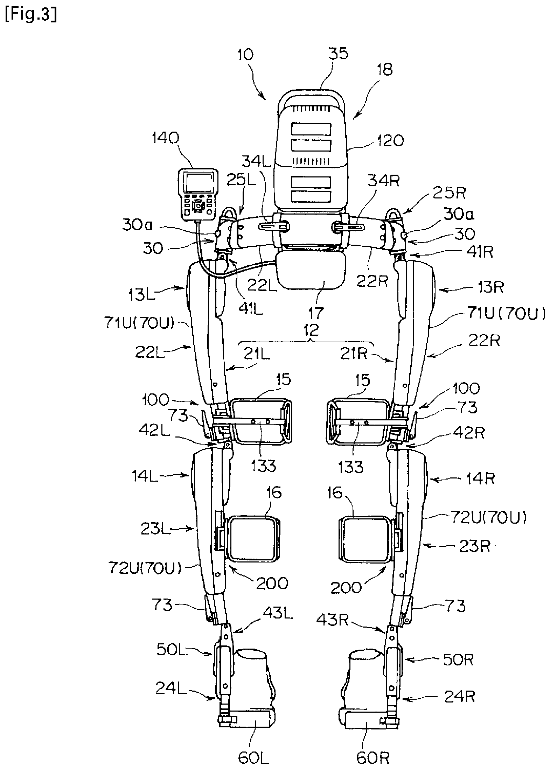

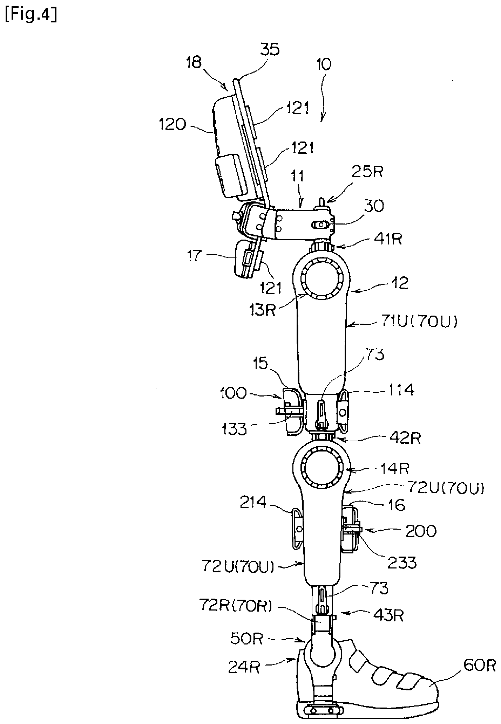

Fig. 1 is an overall perspective view illustrating an example of a wearable action-assistance device (hereunder, referred to as "action-assistance device") of the present invention. Fig. 2 is a front view of the action-assistance device illustrated in Fig. 1. Fig. 3 is a back view of the action-assistance device illustrated in Fig. 1. Fig. 4 is a right side view of the action-assistance device illustrated in Fig. 1.

An action-assistance device 10 includes: a hip frame 11 that is to be worn on the hips of the wearer (not shown; the same applies hereinafter); a lower limb frame 12 that is to be worn on the lower limbs of the wearer; a plurality of drive portions 13L, 13R, 14L and 14R that are provided on the lower limb frame 12 in correspondence with joints of the wearer; cuffs 15L, 15R, 16L and 16R as assistive force exerting members which are attached to the lower limb frame 12 to cause a force from the drive portions 13L, 13R, 14L and 14R to act on the wearer from the front or rear; a control portion 18a that controls the drive portions 13L, 13R, 14L and 14R based on signals that are caused by actions of the wearer; a back face unit 18 in which the control portion 18a is mounted; and an operation unit 140 to be used by an assistant.

### B. Hip Frame

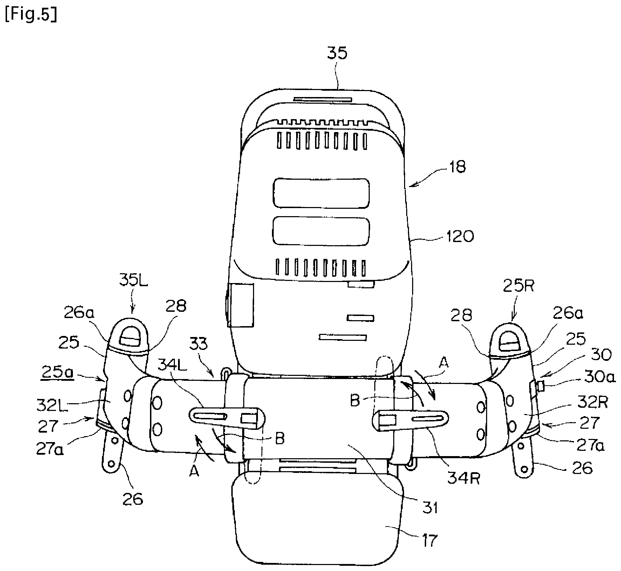

Fig. 5 is a perspective view illustrating a portion constituted by the hip frame 11 and the back face unit 18 of the action-assistance device shown in Fig. 1.

The hip frame 11 is a member which has an approximately "C" shape in plan view and which is open in front to be able to accept the hips of the wearer and surround the hips from the rear to the left and right side thereof, and includes a rear hip frame portion 31 that is positioned at the rear of the wearer, and a left hip frame portion 32L and a right hip frame portion 32R which extend and curves frontward from both ends of the rear hip frame portion 31. The left hip frame portion 32L and the right hip frame portion 32R are connected to the rear hip frame portion 31 via a hip width adjustment mechanism 33. A base of each of the left hip frame portion 32L and the right hip frame portion 32R is inserted into and held inside the rear hip frame portion 31 in a manner in which the respective bases are slidable in the left-right direction.

The hip width adjustment mechanism 33 has lock levers 34L and 34R in the vicinity of both ends of the rear hip frame portion 31. The lock levers 34L and 34R are components for fixing the left hip frame portion 32L and the right hip frame portion 32R to the rear hip frame portion 31 or for releasing a fixed state thereof. The configuration is such that the degree of opening of the hip frame 11 can be adjusted by turning the lock levers 34L and 34R to a side that releases a fixed state thereof (direction of an arrow B in Fig. 5), sliding the left hip frame portion 32L and the right hip frame portion 32R to the left and the right, respectively, relative to the rear hip frame portion 31 to adjust a space between the left and right hip frame portions 32L and 32R, thereafter turning the lock levers 34L and 34R in the opposite direction (direction of an arrow A in Fig. 5) to the direction which releases the fixed state of the lock levers 34L and 34R, and then fixing the lock levers 34L and 34R at rotation limit positions.

### C. Lower Limb Frame

The lower limb frame 12 has a right lower limb frame 21R that is to be worn on a right lower limb of the wearer, and a left lower limb frame 21L that is to be worn on a left lower limb of the wearer. The left lower limb frame 21L and the right lower limb frame 21R are formed to be bilaterally symmetrical.

The left lower limb frame 21L has a left thigh frame 22L that is positioned on the left side of the left thigh of the wearer, a left lower leg frame 23L that is positioned on the left side of the left lower leg of the wearer, and a left foot frame 24L on which the sole of the left foot (if the wearer is wearing shoes, the sole of the shoe on the left side) of the wearer is placed. The left lower limb frame 21L is connected to a tip portion of the left hip frame portion 32L via a hip portion connection mechanism 25L.

The right lower limb frame 21R has a right thigh frame 22R that is positioned on the right side of the right thigh of the wearer, a right lower leg frame 23R that is positioned on the right side of the right lower leg of the wearer, and a right foot frame 24R on which the sole of the right foot (if the wearer is wearing shoes, the sole of the shoe on the right side) of the wearer is placed. The right lower limb frame 21R is connected to a tip portion of the right hip frame portion 32R via a hip portion connection mechanism 25R.

A lower limb frame support portion 25 having a straight pipe structure that is opened at the top and bottom is formed at a tip portion of the left hip frame portion 32L and a tip portion of the right hip frame portion 32R. The hip portion connection mechanisms 25L and 25R each have a spindle 26 that is inserted through the respective lower limb frame support portions 25. The left lower limb frame 21L and the right lower limb frame 21R are connected to a lower end portion of the respective spindles 26. A circular ring-shaped flange portion 26a is formed at an upper end portion of the spindle 26. A load that is applied to the spindle 26 is supported by engagement of the flange portion 26a with an upper end of the lower limb frame support portion 25. An annular retaining member 27 is fitted to a lower end portion of the lower limb frame support portion 25. A circular ring-shaped flange portion 27a is formed in the retaining member 27, and the flange portion 27a is engaged with a lower end of the lower limb frame support portion 25. An internal thread portion, not shown, is formed in an inner circumference portion of a support member 27. An external thread portion, not shown, is formed on an outer circumference of an intermediate portion of the spindle 26. A shock absorbing member 28 formed of an elastic body such as rubber is provided between the spindle 26 and the lower limb frame support portion 25 and between the support member 27 and the lower limb frame support portion 25. When the spindle 26 is inserted though the retaining member 27 and screw portions of the spindle 26 and the retaining member 27 are screwed together and fastened, the spindle 26 is elastically held by the hip frame 11 via the shock absorbing member 28 in a manner that allows the spindle 26 to turn about its own axis.

The hip portion connection mechanisms 25L and 25R each have a turn limiting mechanism 30 that limits a turning angle of the spindle 26 to a predetermined angle (for example, degrees). The turn limiting mechanism 30 is composed of a locking protrusion 30a which is provided at an intermediate portion of the spindle 26, and an elongated hole 25a which is provided at an intermediate portion of the lower limb frame support portion 25. The locking protrusion 30a is provided at a right angle with respect to the spindle 26. The elongated hole 25a extends in the circumferential direction of the lower limb frame support portion 25. The locking protrusion 30a is positioned inside the elongated hole 25a, and moves within the elongated hole 25a in association with a turning motion of the spindle 26 with respect to the lower limb frame support portion 25. The turning angle of the spindle 26 is limited to a predetermined angle or less by limiting the range of movement of the locking protrusion 30a to a range from one end of the elongated hole 25a to the other end thereof.

The left lower limb frame 21L and the right lower limb frame 21R are respectively fixed to the spindles 26 coaxially, and thus the left lower limb frame 21L and the right lower limb frame 21R are connected to the hip frame 11 via the shock absorbing members 28 so as to be rotatable around the respective long axes thereof. Further, by limiting the turning angles of the spindles 26 to a predetermined angle or less, the turning angles of the left lower limb frame 21L and the right lower limb frame 21R with respect to the hip frame 11 are limited to a predetermined angle or less.

Drive portions (hereunder, referred to as "hip-joint drive portions") 13L and 13R having a bearing structure are provided at an upper end portion of the left thigh frame 22L and an upper end portion of the right thigh frame 22R, respectively. A drive motor, not shown, that serves as a source of power is contained inside each of the hip-joint drive portions 13L and 13R. The hip-joint drive portions 13L and 13R can be adjusted to heights that match the hip joints of the wearer. The hip-joint drive portions 13L and 13R each have a fixing element and a rotary element that rotates with respect to the fixing element. By fastening the fixing element of the hip-joint drive portion 13L to the hip portion connection mechanism 25L side and fastening the rotary element to the left thigh frame 22L side, the left thigh frame 22L is pivotally connected to the hip portion connection mechanism 25L such that the left thigh frame 22L is movable backward and forward. By fastening the fixing element of the hip-joint drive portion 13R to the hip portion connection mechanism 25R side and fastening the rotary element to the right thigh frame 22R side, the right thigh frame 22R is pivotally connected to the hip portion connection mechanism 25R such that the right thigh frame 22R is movable backward and forward.

Variable shape portions 41L and 41R that have a hinge mechanism which rotates to the left and right are interposed between the fixing elements of the hip-joint drive portions 13L and 13R and the hip portion connection mechanisms 25L and 25R. The left thigh frame 22L and the right thigh frame 22R are respectively rotatable to the left and right (direction of arrow A in Fig. 2) with respect to the hip portion connection mechanisms 25L and 25R.

Drive portions (hereunder, referred to as "knee-joint drive portions") 14L and 14R having a bearing structure are provided at an upper end portion of the left lower leg frame 23L and an upper end portion of the right lower leg frame 23R, respectively. A drive motor, not shown, that serves as a source of power is contained inside each of the knee-joint drive portions 14L and 14R. The knee-joint drive portions 14L and 14R can be adjusted to heights that match the knee joints of the wearer. The knee-joint drive portions 14L and 14R each have a fixing element and a rotary element that rotates with respect to the fixing element. By fastening the fixing element of the knee-joint drive portion 14L to the left thigh frame 22L side and fastening the rotary element to the left lower leg frame 23L side, the left lower leg frame 23L is pivotally connected connected to the left thigh frame 22L such that the left lower leg frame 23L is movable backward and forward. Further, by fastening the fixing element of the knee-joint drive portion 14R to the right thigh frame 22R side and fastening the rotary element to the right lower leg frame 23R side, the right lower leg frame 23R is pivotally connected to the right thigh frame 22R such that the right lower leg frame 23R is movable backward and forward.

Variable shape portions 42L and 42R that have a hinge mechanism which rotates to the left and right are interposed between the fixing elements of the knee-joint drive portions 14L and 14R and the left thigh frame 22L and right thigh frame 22R. The left lower leg frame 23L and the right lower leg frame 23R are respectively rotatable to the left and right (direction of arrow B in Fig. 2) with respect to the left thigh frame 22L and the right thigh frame 22R.

Foot joint mechanisms 50L and 50R each having a shaft bearing structure are interposed between the lower end of the left lower leg frame 23L and the upper end of the left foot frame 24L, and between the lower end of the right lower leg frame 23R and the upper end of the right foot frame 24R, respectively. The left foot frame 24L and the right foot frame 24R are connected to the left lower leg frame 23L and the right lower leg frame 23R, respectively, in a manner that enables rotation of the left foot frame 24L and the right foot frame 24R to the front and rear. Shoes 60L and 60R into which the feet of the wearer are inserted are fixed on the inner side of the left foot frame 24L and the inner side of the right foot frame 24R.

The foot joint mechanisms 50L and 50R are provided so as to be positioned at a side of the foot joints of the wearer. The angles of the shoes 60L and 60R with respect to the floor surface change in accordance with the walking motion of the wearer as a result of the foot joint mechanisms 50L and 50R rotating in the same manner as the foot joints of the wearer. The foot joint mechanisms 50L and 50R each contain therein an elastic member such as a helical spring, and are urged by an elastic force of the elastic member so that the tip sides of the shoes 60L and 60R do not droop downward.

Variable shape portions 43L and 43R each having a hinge mechanism which rotate to the left and right are interposed between the foot joint mechanisms 50L and 50R and the left lower leg frame 23L and the right lower leg frame 23R, respectively. The left foot frame 24L and the right foot frame 24R are each rotatable to the left and right (direction of arrow C in Fig. 2) with respect to the left lower leg frame 23L and the right lower leg frame 23R.

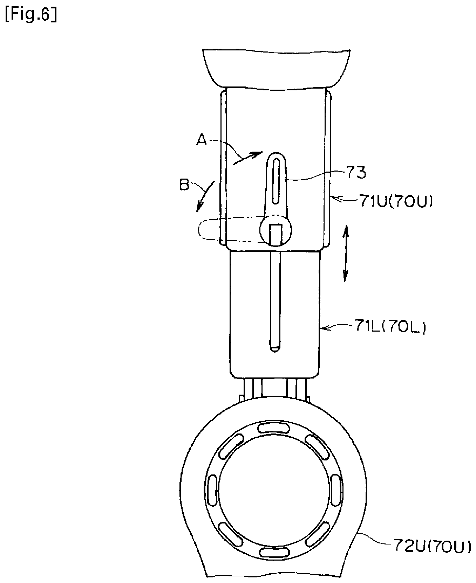

The left thigh frame 22L and the right thigh frame 22R are respectively constituted by a thigh frame main body 71U (upper-side element 70U) and a slide member 71L (lower-side element 70L). The left lower leg frame 23L and the right lower leg frame 23R are respectively constituted by a lower leg frame main body 72U (upper-side element 70U) and a slide member 72L (lower-side element 70L). The upper-side element 70U and the lower-side element 70L are configured to be slidable with respect to each other in a longitudinal direction. As shown in Fig. 6, a lock lever 73 for fixing the two elements 70U and 70L to each other or releasing a fixed state therebetween is provided on the upper-side element 70U. The configuration is such that the lengths of the respective frames 22L, 22R, 24L and 24R can be respectively adjusted by rotating the lock lever 73 to a side that releases a fixed state (direction of arrow B in Fig. 6) and sliding the two elements 70U and 70L with respect to each other to adjust the relative position between the two elements, and thereafter rotating the lock lever 73 in the direction that is opposite (direction of arrow A in Fig. 6) to the direction that released the fixed state, and fixing the lock lever 73 at the rotation limit position.

Each thigh frame main body 71U has a thick portion on an upper side (hereunder, described as "upper part"), and a thin portion on a lower side, that is, a portion that houses the lower-side element 70L (hereunder, described as "lower part"). Variable shape portions 44L, 44R, 45L and 45R each having a hinge mechanism which rotates to the left and right are interposed between the upper part and lower part of each of the thigh frame main bodies 71U. Each lower part is rotatable to the left and right (directions of arrows D and E in Fig. 2) with respect to the corresponding upper part of the thigh frame main bodies 71U.

### D. Cuffs

The cuffs 15 and 16 are provided one each on the left thigh frame 22L, the right thigh frame 22R, the left lower leg frame 23L and the right lower leg frame 23R, respectively.

### D1. Thigh Cuffs

The respective cuffs (hereunder, described as "thigh cuff") 15 provided on the left thigh frame 22L and the right thigh frame 22R are supported by respective thigh cuff support mechanisms 100 that are attached to a lower end portion of the thigh frame main body 71U, and are provided at a center portion in the vertical direction of the thigh region of the wearer or in the vicinity thereof. Each thigh cuff 15 has a mounting face which is curved in an arc shape that can be arranged so as to fit to a thigh of the wearer. A fitting member is attached to the mounting face of each thigh cuff 15 so that the mounting face can closely contact the corresponding thigh of the wearer without a gap therebetween.

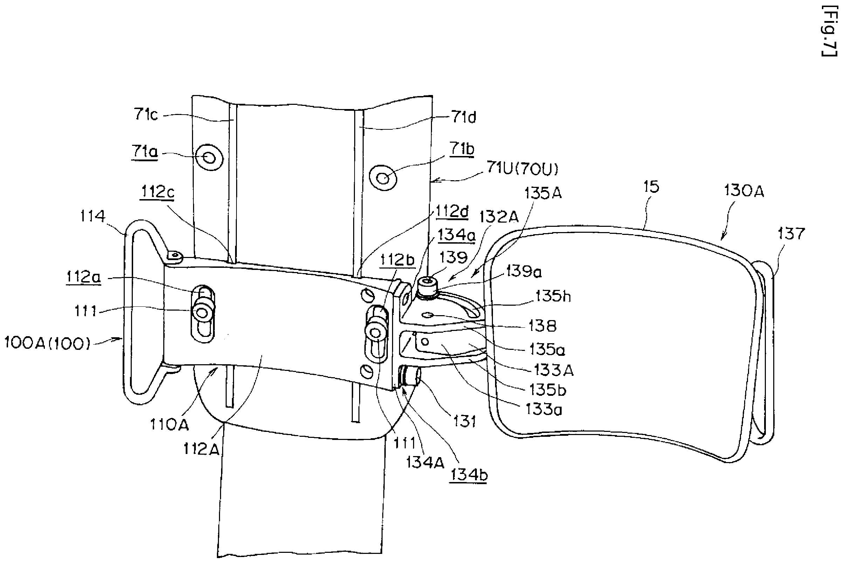

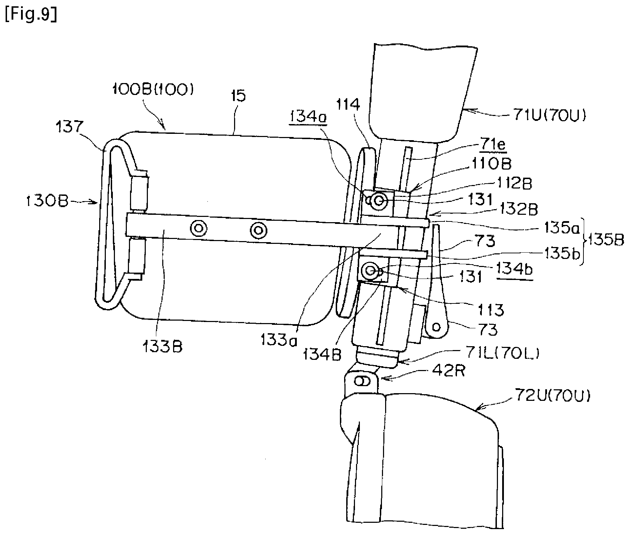

Fig. 7 and Fig. 9 are perspective views illustrating a portion of the action-assistance device shown in Fig. 1 at which the thigh cuff 15 and the thigh cuff support mechanism 100 (100A and 100B) are provided. There are two forms of attaching the thigh cuff 15, namely, a form in which the thigh cuff 15 is attached to the upper part of the thigh frame main body 71U by a first thigh cuff support mechanism 100A as shown in Fig. 7, and a form in which the thigh cuff 15 is attached to the lower part of the thigh frame main body 71U by a second thigh cuff support mechanism 100B as shown in Fig. 9.

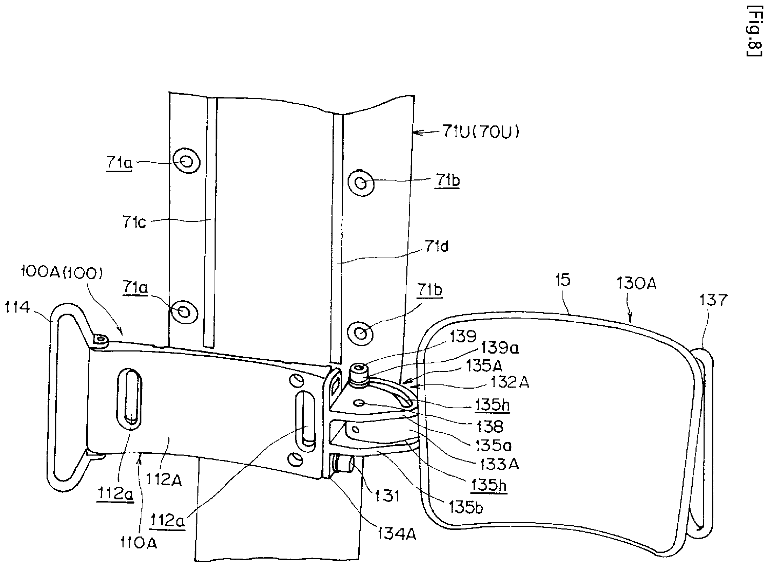

Fig. 8 is a perspective view illustrating a state immediately before attaching (or immediately after detaching) the first thigh cuff support mechanism 100A to the upper part of the thigh frame main body 71U. The first thigh cuff support mechanism 100A has a fixing member 110A that is fixed to the upper part of the thigh frame main body 71U, and a cuff support member 130B that is connected to the fixing member 110A.

The fixing member 110A has a plate-shaped fixing portion 112A, which is elongated transversely, that is fixed by fastening with a screw 111 to an inner face of the thigh frame main body 71U. Elongated holes 112a and 112b that extend vertically are respectively formed in the vicinity of the front and back ends the fixing portion 112. Screw-holes 71a and 71b are formed at the front and back in an inner face of the upper part of the thigh frame main body 71U in correspondence with the positions of the elongated holes 112a and 112b at the front and back of the fixing portion 112A. Two each of the screw holes 71a and 71b are provided such that they are spaced apart in the vertical direction. The first thigh cuff support mechanism 100A can be attached at any position in the vertical direction by inserting the screws 111 into the elongated holes 112a and 112b at the front and back of the fixing portion 112A, and inserting the screws 111 into either of the upper and lower screw holes 71a and 71b and tightening the screws 111. Further, in a state in which the screws 111 are loose, it is possible to finely adjust the height position of the first thigh cuff support mechanism 100A by vertically moving the positions of the elongated holes 112a and 112b relative to the screws 111. Two guide protrusions 71c and 71d which are parallel to each other and which extend vertically are formed in the inner face of the upper part of the thigh frame main body 71U. Two guide grooves 112c and 112d which fit over the guide protrusions 71c and 71d are formed in the fixing portion 112A. Wobbling of the fixing portion 112A with respect to the thigh frame main body 71U is prevented by fitting of the guide protrusions 71c and 71d of the fixing portion 112 over the guide protrusions 71c and 71d of the thigh frame main body 71U.

The first thigh cuff support mechanism 100 can be easily detached from the thigh frame main body 71U by unfastening the screws 111 and 111 at the front and back that fix the fixing member 110A of the first thigh cuff support mechanism 100 to the inner face of the thigh frame main body 71U. Further, by inverting the front and rear sides of the first thigh cuff support mechanism 100 and then performing reattachment thereof, the front and back positions of the thigh cuff 15 with respect to the lower limb frame 12 can be changed.

A belt attaching portion 114 having a looped shape for attaching a lower limb belt 81, described later, is provided at one of the end portions at the front and back of the fixing member 110A, and the cuff support member 130 is attached to the other end portion.

The cuff support member 130 has a bracket member 132A that is fastened and fixed by screws 131 to an end face of the fixing member 110A, and an arm member 133A that is rotatably supported in an approximately horizontal direction by the bracket member 132A. The cuff 15 is fixed to the arm member 133A. A belt attaching portion 137A having a looped shape for attaching the lower limb belt 81, described later, is provided at a tip portion of the arm member 133A.

The bracket member 132A has a plate-shaped base 134 that is joined to the end face of the fixing member 110A, and a bracket-shaped arm support portion 135 that rotatably supports the arm member 133A. Screw insertion holes 134a and 134b, which extend transversely, are formed in the base 134 in correspondence with positions of tapped holes (not shown) that are formed at two places at the top and bottom of the end face of the fixing member 110A. The arm support portion 135A has a pair of upper and lower flat plate portions 135a and 135b. A base 133a of the arm member 133A is disposed between the two flat plate portions 135a and 135b, and the base 133a and the two flat plate portions 135a and 135b are rotatably connected with respect to each other by a spindle 138 that passes therethrough in the vertical direction.

Guide holes 135h and 135h each having an arc shape which has the position of the spindle 138 as a center of curvature are formed in the two flat plate portions 135a and 135b, respectively. Screws 139 that are inserted through the guide holes 135h and 135h from the upper side and lower side, respectively, are attached at the top and bottom of the base of the arm member 133A. Head portions 139a of the screws 139 engage with the flat plate portions 135a and 135b. The arm member 133A is rotatable with respect to the bracket member 132A within a range in which the screws 139 are movable inside the guide holes 135h and 135h. It is possible to adjust and fix an angle in the horizontal direction of the arm member 133A relative to the bracket member 132A to any angle by tightening the screws 139.

Further, the bracket member 132A is configured such that, by loosening the upper and lower screws 131 fixing the base 134 of the bracket member 132A to the end face of the fixing member 110A, its angle can be changed in the left-right direction with respect to the fixing member 110A within an allowable range of the screw insertion holes 134a that are formed in a transversely elongated shape, and by tightening the screws 131, it can be fixed with its angle being adjusted in the left-right direction of the arm member 133A with respect to the fixing member 110A to any angle (see Fig. 9).

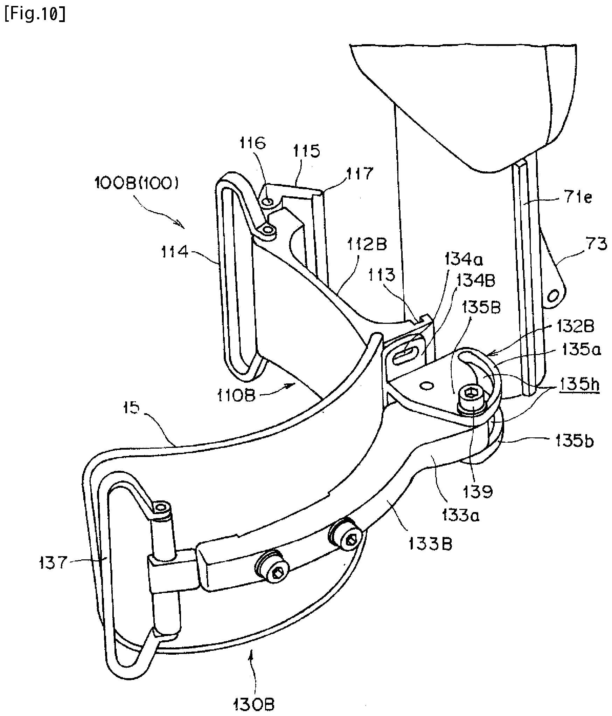

Fig. 10 is a perspective view illustrating a state immediately before attaching (or immediately after detaching) the second thigh cuff support mechanism 100B to the lower part of the thigh frame main body 71U. The second thigh cuff support mechanism 100B has a fixing member 110B that is fixed to the lower part of the thigh frame main body 71U, and the cuff support member 130B that is connected to the fixing member 110B.

The fixing member 110B has a plate-shaped fixing portion 112B which is transversely elongated that is fixed to an inner face of the lower part of the thigh frame main body 71U. A locking groove 113 that extends vertically is formed in one end portion in the front-rear direction of the fixing portion 112B. A lock member 115 having an approximately "L" shape in a plan view is provided at the other end portion in the front-rear direction of the fixing portion 112B. A base end portion of the lock member 115 is connected to the fixing portion 112B in a rotatable condition in the horizontal direction via a rotary shaft 116. A locking claw 117 that extends vertically is formed at a tip portion of the lock member 115.

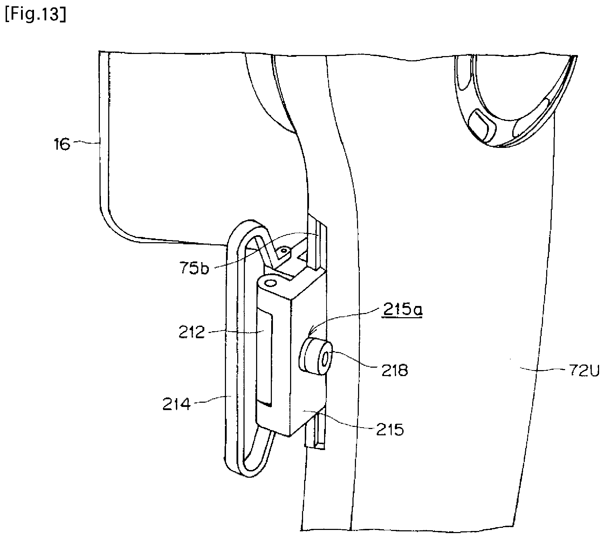

The lock member 115 is fixed in a non-rotatable condition to the fixing portion 112B by placing the locking claw 117 in a state facing the locking groove 113 (a locked state), and fastening to the fixing portion 112B with a screw, not shown (see reference numeral 213 in Fig. 13).

Linear guide protrusions 71e and 71f (71f is not shown in the drawings) that extend vertically are formed in a front face and a rear face of the lower part of the thigh frame main body 71U. The guide protrusions 71e and 71f are formed so that the locking groove 113 of the fixing member 110B and the locking claw 117 of the lock member 115 can engage therewith, respectively. In a state in which the locking groove 113 and the locking claw 117 are locked in the guide protrusions 71e and 71f at the front and back of the lower part of the thigh frame main body 71U, by placing the lock member in a locked state and fixing the lock member with a screw (see reference numeral 213 in Fig. 13), the second thigh cuff support mechanism 100B is attached to the lower part of the thigh frame main body 71U in a manner such that the second thigh cuff support mechanism 100B embraces the inner face thereof. Wobbling of the fixing member 110B with respect to the thigh frame main body 71U is prevented by the locking groove 113 and the locking claw 117 that are provided at the front and back of the fixing member 110B fitting onto the guide protrusions 71e and 71f at the front and back of the lower part of the thigh frame main body 71U.

The cuff support member 130B is attached to the end portion at which the locking groove 113 of the fixing member 110B is provided. The belt attaching portion 114 having a looped shape for attaching the lower limb belt 81, described later, is provided at the end portion at which the lock member 115 of the fixing member 110B is provided.

The cuff support member 130B has a bracket member 132B that is fastened and fixed by the screws 131 to an end face of the fixing member 110B, and an arm member 133B that is rotatably supported in an approximately horizontal direction by the bracket member 132B. The cuff 15 is fixed to the arm member 133B. A belt attaching portion 137 having a looped shape for attaching the lower limb belt 81, described later, is provided at a tip portion of the arm member 133B.

The bracket member 132B has a plate-shaped base 134B that is joined to the end face of the fixing member 110B, and a bracket-shaped arm support portion 135B that rotatably supports the arm member 133B. Horizontally long screw insertion holes 134a are formed in the base 134B in correspondence with positions of tapped holes formed at two places at the top and bottom of the end face of the fixing member 110B. The arm support portion 135B has a pair of upper and lower flat plate portions 135a and 135b. A base 133a of the arm member 133B is disposed between the two flat plate portions 135a and 135b, and the base 133a and the two flat plate portions 135a and 135b are rotatably connected with respect to each other by a spindle, not shown, that passes therethrough in the vertical direction.

Guide holes 135h each having an arc shape which have the position of the spindle as a center of curvature are formed in the two flat plate portions 135b, respectively. Screws 139 that are inserted through the guide holes 135h from the upper side and lower side, respectively, are attached at the top and bottom of the base of the arm member 133B. The arm member 133B is swingable with respect to the bracket member 132B within a range in which the screws thereof are movable inside the guide holes 135h. It is possible to adjust and fix an angle in the horizontal direction of the arm member 133B relative to the bracket member 132B to any angle by tightening the screws 139.

Further, the bracket member 132B is configured such that, by loosening the upper and lower screws 131 fixing the base 134B of the bracket member 132B to the end face of the fixing member 110B, its angle can be changed in the left-right direction with respect to the fixing member 110B within an allowable range of the screw insertion holes 134a that are formed in a transversely elongated, and by tightening the screws 131, it can be fixed with its angle being adjusted in the left-right direction of the arm member 133B with respect to the fixing member 110B to any angle.

Further, it is possible to adjust the height position at which the second thigh cuff support mechanism 100B is attached to the thigh frame main body 71U by loosening the screw fixing the lock member of the second thigh cuff support mechanism 100B and sliding the fixing portion 112B vertically along the guide protrusions 71e and 71f.

Furthermore, it is possible to easily detach the second thigh cuff support mechanism 100B from the thigh frame main body 71U by unfastening the screw fixing the lock member thereof. Further, by inverting the front and rear sides of the second thigh cuff support mechanism 100B and then performing reattachment thereof, the front and back positions of the thigh cuff 15 with respect to the lower limb frame 12 can be changed.

### D2. Lower-leg Cuff

The cuffs (hereunder, referred to as "lower-leg cuffs") 16 provided on the left lower leg frame 23L and the right lower leg frame 23R are supported by lower-leg cuff support mechanisms 200 that are attached to the upper-side elements 72U (70U). Each lower-leg cuff 16 is provided at a center portion in the vertical direction of a lower-leg region of the wearer or the vicinity thereof. Each lower-leg cuff 16 has a mounting face which is curved in an arc shape that can be arranged so as to fit to a lower leg of the wearer. A fitting member is attached to the mounting face of each lower-leg cuff 16 so that the mounting face can closely contact the corresponding lower leg of the wearer without a gap therebetween.

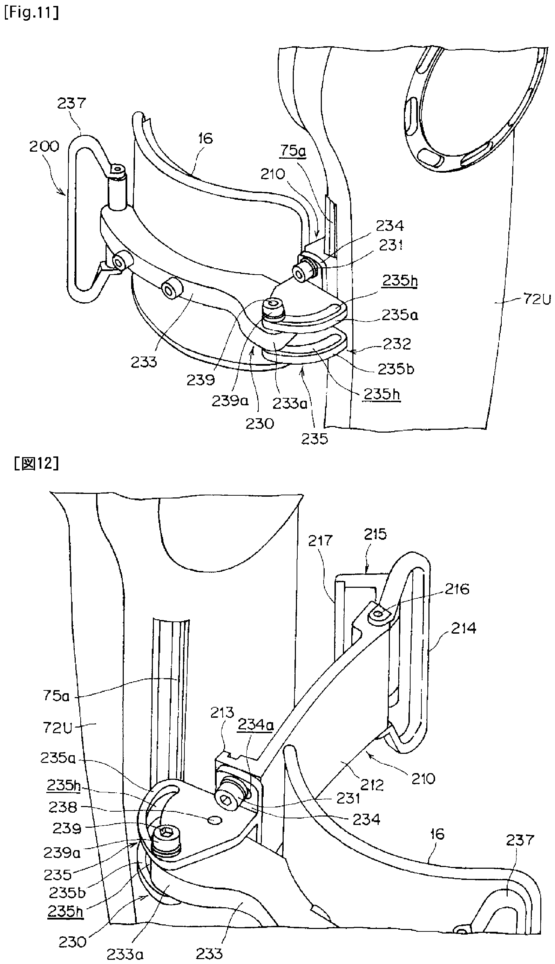

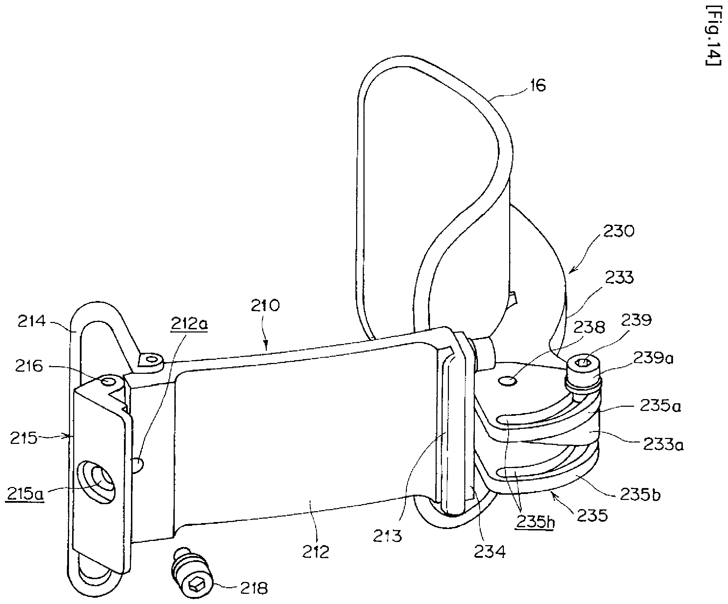

Fig. 11 is a perspective view illustrating a portion of the action-assistance device 10 at which the lower-leg cuff 16 and the lower-leg cuff support mechanism 200 are provided. Fig. 12 is a perspective view illustrating a state when attaching the lower-leg cuff support mechanism 202 to the lower leg frame main body 72U. Fig. 13 is a perspective view of a principal portion which illustrates a state in which the lower-leg cuff support mechanism 200 is attached. Fig. 14 is a perspective view of the lower-leg cuff support mechanism 200.

The lower-leg cuff support mechanism 200 has a fixing member 210 that is fixed to the lower leg frame main body 72U, and a cuff support member 230 that is connected to the fixing member 210.

The fixing member 210 has a plate-shaped fixing portion 212 which is horizontally long that is fixed to an inner face of the lower leg frame main body 72U. A first locking claw 213 that extends vertically is formed at one end portion in the front-rear direction of the fixing portion 212. A lock member 215 having an approximately "L" shape in plan view is provided at the other end portion in the front-rear direction of the fixing portion 212. A base end portion of the lock member 215 is connected to the fixing portion 212 in a horizontally rotatable manner via a rotary shaft 216. A second locking claw 217 that extends vertically is formed at a tip portion of the lock member 215.

A screw insertion hole 215a through which a screw 218 is to be inserted is formed at a center portion in the vertical direction of the lock member 215. A screw hole 212a in which the screw 218 is to be fastened is formed in the fixing portion 212 at a position which overlaps with the screw insertion hole 215a at a time that the second locking claw 217 of the lock member 215 is placed in a state facing the first locking claw 213 (locked state). The lock member 215 is fixed in a non-rotatable manner to the fixing portion 212 by placing the lock member 215 in a locked state and tightening the screw 218.

Linear locking grooves 75a and 75b that extend vertically are formed in a front face and a rear face of the lower leg frame main body 72U. The locking grooves 75a and 75b are formed in the vicinity of a front edge and the vicinity of a rear edge of the inner face of the lower leg frame main body 72U so that the first locking claw 213 of the fixing member 110 and the second locking claw 217 of the lock member 215 can respectively engage therewith. In a state in which the first locking claw 213 and the second locking claw 217 are locked in the locking grooves 75a and 75b at the front and back of the lower leg frame main body 72U, by placing the lock member 215 in a locked state and fixing the lock member 215 with the screw 218, the lower-leg cuff support mechanism 200 is attached to the lower leg frame main body 72U in a manner such that the lower-leg cuff support mechanism 200 embraces the inner face thereof. Wobbling of the fixing member 110 with respect to the lower leg frame main body 72U is prevented by the first locking claw 213 and the second locking claw 217 that are provided at the front and back of the fixing member 110 fitting into the locking grooves 75a and 75b at the front and back of the lower leg frame main body 72U.

The cuff support member 230 is attached to the end portion at which the first locking claw 213 of the fixing member 210 is provided. A belt attaching portion 214 having a looped shape for attaching the lower limb belt 81 that is described later is provided at the end portion at which the lock member 215 of the fixing member 210 is provided.

The cuff support member 230 has a bracket member 232 that is fastened and fixed by screws 231 to an end face of the fixing member 210, and an arm member 233 that is rotatably supported in an approximately horizontal direction by the bracket member 232. The cuff 16 is fixed to the arm member 233. A belt attaching portion 237 having a looped shape for attaching the lower limb belt 81, described later, is provided at a tip portion of the arm member 233.

The bracket member 232 has a plate-shaped base 234 which is to be joined to the end face of the fixing member 210, and a bracket-shaped arm support portion 235 that rotatably supports the arm member 233. Horizontally long screw insertion holes 234a are formed in the base 234 in correspondence with positions of tapped holes (not shown) formed at two places at the top and bottom of the end face of the fixing member 210. The arm support portion 235 has a pair of upper and lower flat plate portions 235a and 235b. A base 233a of the arm member 233 is disposed between the two flat plate portions 235a and 235b, and the base 233a and the two flat plate portions 235a and 235b are rotatably connected with respect to each other by a spindle 238 that passes therethrough in the vertical direction.

Guide holes 235h and 235h each having an arc shape which have the position of the spindle 238 as a center of curvature are formed in the two flat plate portions 235a and 235b, respectively. Screws 239 that are inserted through the guide holes 235h and 235h from the upper side and lower side, respectively, are attached at the top and bottom of the base of the arm member 133. Head portions 239a of the screws 239 engage with the flat plate portions 235a and 235b. The arm member 233 is rotatable with respect to the bracket member 232 within a range in which the screws 239 are movable inside the guide holes 235h and 235h. It is possible to adjust and fix an angle in the horizontal direction of the arm member 233 relative to the bracket member 232 to any angle by tightening the screws 239.

Further, the bracket member 232 is configured such that, by loosening the upper and lower screws 231 fixing the base 234 to the end face of the fixing member 210, its angle can be changed in the left-right direction with respect to the fixing member 210 within an allowable range of the screw insertion holes 234a that are formed in a transversely elongated shape, and by tightening the screws 231, it can be fixed with its angle being adjusted in the left-right direction of the arm member 233 with respect to the fixing member 210 to any angle.

It is possible to adjust the height position at which the lower-leg cuff support mechanism 200 is attached to the lower leg frame main body 72U by loosening the screw 218 fixing the lock member 215 and sliding the fixing portion 212 vertically along the locking grooves 75a and 75b.

Furthermore, it is possible to easily detach the lower-leg cuff support mechanism 200 from the lower leg frame main body 72U by unfastening the screw 218 fixing the lock member 215. Further, by inverting the front and rear sides of the lower-leg cuff support mechanism 200 and then performing reattachment thereof, the front and back positions of the lower-leg cuff 16 with respect to the lower limb frame 12 can be changed.

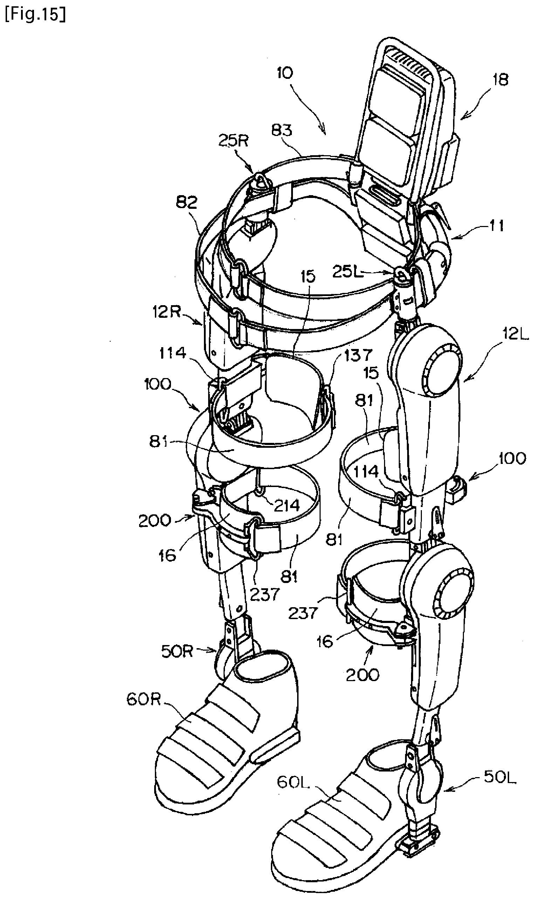

Fig. 15 is a perspective view illustrating a state at a time of wearing the action-assistance device illustrated in Fig. 1.

As shown in Fig. 15, when wearing the action-assistance device 1, the lower limb belts 81 are used for fitting the lower limb frame 12 closely with the thigh regions and the lower-leg regions. Further, trunk portion belts 82 and 83 are used for fitting the hip frame 11 and the back face unit 18 closely with the trunk portion of the wearer.

Each lower limb belt 81 has a flat fastener structure at each of the two end portions thereof. In a state in which the thigh cuffs 15 are disposed on the front side or rear side of the thighs of the wearer, the thigh frames 22L and 22R on the left and right of the lower limb frame 12 can be made to fit closely with the thigh regions of the wearer by connecting both end portions of each lower limb belt 81 to the belt attaching portions 114 and 137 of the corresponding thigh cuff support mechanism 100 and adjusting the degree of tightening of the lower limb belts 81 so that the thigh cuffs 15 closely contact the thighs. Further, in a state in which the lower-leg cuffs 16 are disposed on the front side or rear side of the lower legs of the wearer, the lower leg frames 23L and 23R on the left and right of the lower limb frame 12 can be made to fit closely with the lower-leg regions of the wearer by attaching both end portions of each belt 81 to the belt attaching portions 214 and 237 of the corresponding lower-leg cuff support mechanism 200 and adjusting the degree of tightening of the lower limb belts 81 so that the lower-leg cuffs 16 closely contact the lower legs.

Since the lower limb frame 12 is closely fitted to the lower limbs of the wearer, a driving torque generated at the drive portions 13L, 13R, 14L and 14R can be effectively transmitted as an assistive force to the lower limbs of the wearer via the lower limb frame 12. At such time, forces generated by the left and right hip-joint drive portions 13L and 13R are efficiently transmitted to the thigh regions of the wearer through the thigh cuffs 15 provided on the left and right thigh frames 22L and 22R. Further, forces generated by the left and right knee-joint drive portions 14L and 14R are efficiently transmitted to the lower-leg regions of the wearer through the lower-leg cuffs 16 provided on the left and right lower leg frames 23L and 23R.

### E. Back Face Unit

The back face unit 18 is attached to a center portion in the horizontal direction of the rear hip frame portion 31. The back face unit 18 has a pipe frame 35 that is formed by bending a pipe material in an approximately rectangular ring shape. The pipe frame 35 curves somewhat rearward at an intermediate portion in a vertical direction thereof, and a portion on a lower side than the curved portion thereof is brought into contact with the front face of the frame portion 31 and is fixed to the rear hip frame portion 31 by a fastening metal fitting 36. A switch box 17 in which a power switch is provided is attached to a lower part of the pipe frame 35, and a power supply portion 120 is attached to an upper part of the pipe frame 35. Shock absorbing pads 121 are affixed to the front face of the control portion 18a and the power supply portion 120, that is, portions facing the wearer.

### F. Operation Unit

### (Description of Operation Unit 140)

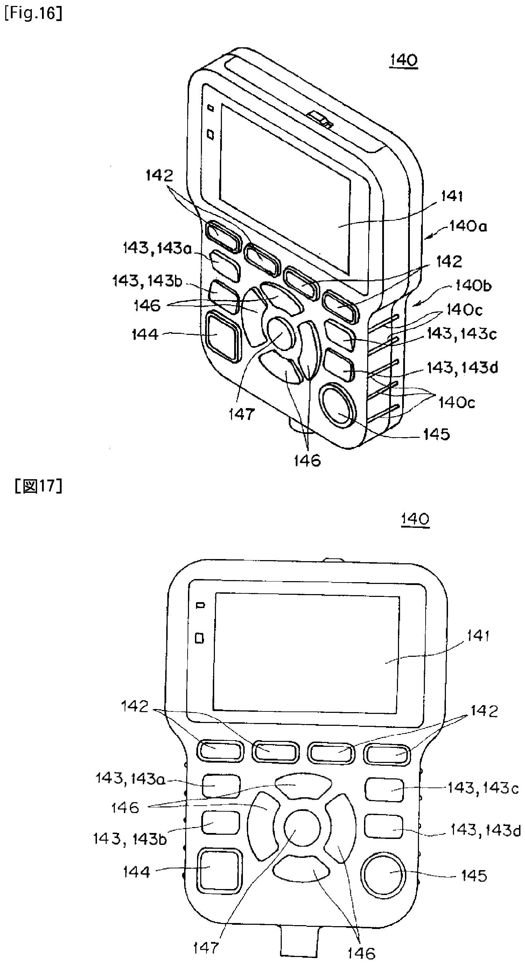

As shown in Fig. 16, the operation unit 140 has a display 141, task selection buttons 142, joint buttons 143, a stop button 144, a start button 145, arrow keys 146, and a confirmation operation button 147. As shown in the perspective view in Fig. 17, in the operation unit 140, a casing upper part 140a in which the display 141 is disposed has a broader shape relative to a casing lower part 140b in which the various buttons (142 to 147) are disposed. In other words, by making the width of the casing lower part 140b narrower than the width of the casing upper part 140a in this way, the casing lower part 140b functions as a handle of the operation unit 140 for the assistant. In addition, since a plurality of grooves 140c are provided in the side faces of the casing lower part 140b, fingers of the assistant catch in the grooves 140c when the assistant grasps the casing lower part 140b of the operation unit 140. That is, the operation unit 140 can be prevented from slipping out from the hand of the assistant.

The display 141 displays settings and the operating state of the action-assistance device 10. The task selection buttons 142 include a plurality of buttons for classifying respective action patterns of the wearer such as a standing-up action, a walking action or a sitting down action as tasks, and for accepting operations that select the respective tasks. When a single task is selected by the task selection buttons 142, as described later, the relevant task is displayed on task indicators 153 and 171c, and control based on the selected task by the control portion 18a is performed.

As shown in Fig. 17, the joint buttons 143 are operation buttons for displaying assistive force settings and operating states relating to each joint on the display 141.

Specifically, the joint buttons 143 are a left hip joint button 143a, a left knee joint button 143b, a right hip joint button 143c and a right knee joint button 143d. The respective joint buttons are disposed on the left upper side, left lower side, right upper side and right lower side relative to the center of the casing lower part 140b of the operation unit 140. In other words, the arrangement of the joint buttons 143a to 143d corresponds to the positions of the respective joints of the wearer when the assistant views the operation unit 140 while assisting the wearer from the rear side. By adopting this arrangement, it is possible for the assistant to intuitively operate the buttons without any mistakes.

The stop button 144 is disposed, for example, on a lower side of the right knee joint button 143d, and is used for performing an operation to stop assistance by the action-assistance device 10. The start button 145 is disposed, for example, on a lower side of the left knee joint buttons 143b, and is used for performing an operation to start assistance by the action-assistance device 10. By disposing the stop button 144 and the start button 145 on the left and right sides with a large space therebetween across the arrow keys 146, erroneous operation of the device can be prevented.

The arrow keys 146 are disposed, for example, on the center side of the casing lower part 140b relative to the joint buttons 143, and are used for moving a cursor position that is displayed on the display 141 or to increase or decrease an adjustable value using the cursor. The confirmation operation button 147 is disposed at the center position of the casing lower part 140b, and is used for confirming an input value that is input utilizing another button and for performing an operation that switches the display screen.

### (Description of Display on Display 141)

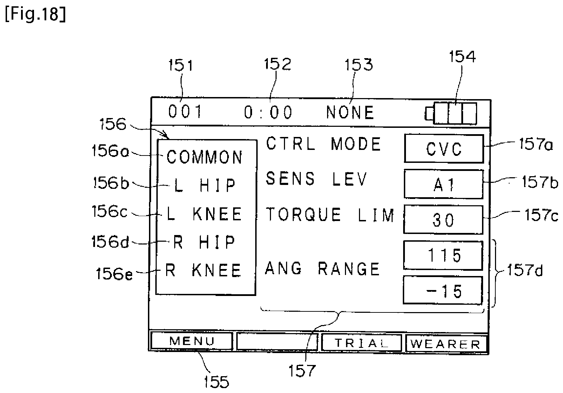

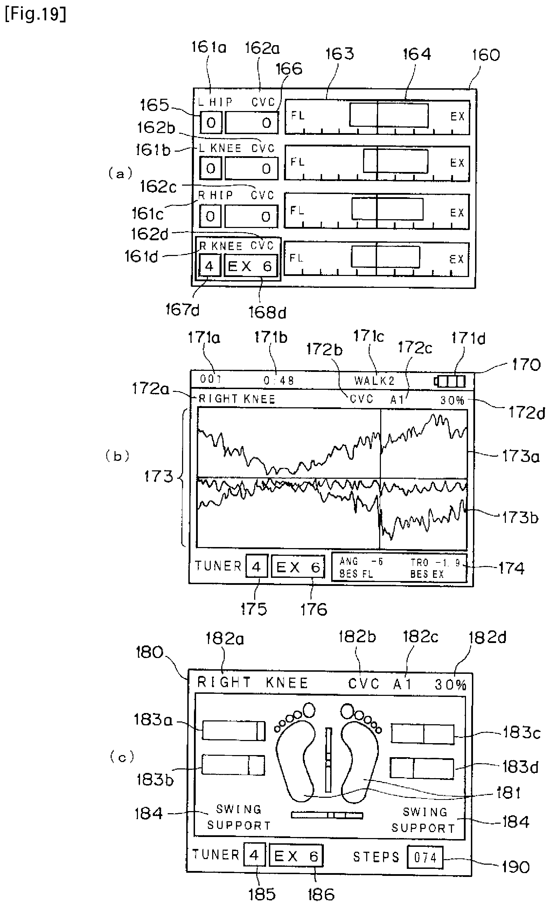

An example of the screen display of the display 141 in the above described operation unit 140 will now be described using Fig. 18 and Figs. 19A to 19C. Fig. 18 illustrates an example of a settings screen 150 for performing settings of the action-assistance device 10. Figs. 19A to 19C illustrate examples of a screen 160, a screen 170 and a screen 180 during assistance that display operating states and the like. Figs. 19A to 19C are views that illustrate three display pattern examples, and these screens are switched in sequence by performing an operation such as pressing the confirmation operation button 147 (see Fig. 16 and Fig. 17).

### (Description of Settings Screen)

As shown in Fig. 18, as menu display items, the settings screen 150 includes: a wearer ID 151 indicating that information is the one that the wearer has individually set; an assistance elapsed time 152 that shows a time period since the device was started; a task indicator 153 that displays the current task; a residual battery amount indicator 154 that shows the residual amount of the device battery; and a shortcut choice 155 that shows a "Menu" button or the like. The settings screen 150 also includes, as screen display items, a list of regions 156 and a settings editing items field 157.

Among these, the current task that has been selected by means of the task selection buttons 142 is displayed on the task display 153. For example, in a case where "WALK" is selected by means of the task selection buttons 142, "WALK" is displayed in the task display 153. Further, in a case where, as described later, a control mode is set in which a control portion 18a determines the posture of the wearer based on various detection signals and switches the task, each time a task is switched in accordance with the determination result obtained by the control portion 18a, the task display 153 displays the task that is switched to.

The list of region 156 includes, as items to be chosen, in order from the top, a "COMMON" button 156a for performing settings associated with the overall system, an "L HIP" button 156b for performing settings associated with assistance by the drive portion 71L for the left hip joint, an "L KNEE" button 156c for performing settings associated with assistance of by drive portion 72L for the left knee joint, an "R HIP" button 156d, for performing settings associated with assistance by the drive portion 71R for the right hip joint, and an "R KNEE" button 156e for performing settings associated with assistance by the drive portion 72R for the right knee joint. Although each of the "L HIP" button 156b, the "L KNEE" button 156c, the "R HIP" button 156d and the "R KNEE" button 156e can also be selected by moving a selection button upward/downward by performing an upward/downward operation of the aforementioned arrow keys 146, optimally the aforementioned buttons can be directly selected by selecting the joint buttons 143a to 143d.

The settings editing items field 157 includes, in order from the top: a "CTRL MODE" item 157a for setting a control mode that performs assistance of the wearer; a "SENS LEV" item 157b for setting a sensitivity level that selects a filter that processes biopotential signals and an amplification factor; a "TORQUE LIM" item 157c for setting an output upper limit of assistance of the wearer; and an "ANG RANGE" item 157d for setting an assistance angle range (°) with respect to flexion angles (initial values: HIP = 115°, KNEE = 115°) and extension angles (initial values: HIP = -15°, KNEE = 0°).

These settings editing items are edited by the following procedure. That is, when setting any drive portion among the drive portion 71L for the left hip joint, the drive portion 72L for the left knee joint, the drive portion 71R for the right hip joint and the drive portion 72R for the right knee joint, the assistant first selects the relevant joint button among the joint buttons 143a to 143d that correspond to the respective drive portions on the operation unit 140. For example, when the assistant selects the joint button 143a, as shown in Fig. 18, the "L HIP" button 156b is displayed in a highlighted manner in gray, and at the same time, one of the setting items in the settings editing items field 157 is displayed in a highlighted manner in gray (in this case, the "CTRL MODE" item 157a), and a setting operation of the settings editing items field 157 is enabled. In this state, the assistant selects an item from 157a to 157d in the settings editing items field 157 by performing an upward/downward operation of the arrow keys 146, and edits the setting of one of the items.

### (Description of Operation Screens During Assistance)

As shown in Fig. 19A, the screen 160 during assistance includes, as indicator items, a joint name indicator 161, a control mode indicator 162, a flexor biopotential signal level indicator 163, an extensor biopotential signal level indicator 164, a torque tuner setting value indicator 165, and a balance tuner setting value indicator 166.

The joint name indicator 161 includes an "L HIP" indicator 161a that shows an assistance setting of the drive portion 71L for the left hip joint, an "L KNEE" indicator 161b that shows an assistance setting of the drive portion 72L for the left knee joint, an "R HIP" indicator 161c that shows an assistance setting of the drive portion 71R for the right hip joint, and an "R KNEE" indicator 161d that shows an assistance setting of the drive portion 72R for the right knee joint.

The control mode indicator 162 is provided with indicators 162a to 162d that correspond to the indicators 161a to 161d of the joint name indicator 161. In the flexor biopotential signal level indicator 163 also, indicators 163a to 163d are provided that correspond to the indicators 161a to 161d of the joint name indicator 161, with the level values being displayed so as to increase or decrease in the leftward direction in the drawing in a bar graph format. Further, in the extensor biopotential signal level indicator 164, indicators 164a to 164d are provided that correspond to the indicators 161a to 161d of the joint name indicator 161, with the level values thereof being displayed so as to increase or decrease in the rightward direction in the drawing in a bar graph format.

The screen 170 during assistance is a display that, as shown in Fig. 19B, is transitioned to in a case where any one indicator among the indicators 161a to 161d of the joint name indicator 161 shown on the screen in Fig. 19A is selected. In this case, a state is shown in which the displaying of "R KNEE" of the indicator 161d on the top row in Fig. 19A is selected. Similarly to the screen illustrated in Fig. 18, screen information is displayed for the following items as menu display items on the screen 170 during assistance, namely: a wearer ID 171a indicating that information is the one that the wearer has individually set; an assistance elapsed time 171b that shows a time period since the device was started; a task indicator 171c (WALK2) that displays the current task; and a residual battery amount indicator 171d that shows the residual amount of the device battery.

Further, on the second row from the top of the screen 170, an indicator 172a (RIGHT KNEE) of the joint name that is selected, a control mode indicator 172b (CVC), a sensitivity level indicator 172c (A1), and a torque limit 172d (30%) are displayed. Furthermore, an assistance angle range 173a and waveforms 173b are displayed in a graph indicating region 173 in the center of the screen. The waveforms 173b show waveforms for an angle, an assistance torque, a biopotential signal of a flexor and a biopotential signal of an extensor in the form of different colored polygonal lines. In addition, on the lower right of the screen 170, an indicator 174 is displayed which shows an angle (°) and an assistance torque (Nm) by means of numerical values, while on the lower left of the screen 170, a torque tuner setting value 175 of the selected joint and a balance tuner setting value 176 of the selected joint are displayed.

As shown in Fig. 19C, a screen 180 during assistance is a display screen that is switched to by pressing the confirmation operation button 147 (see Fig. 16 and Fig. 17) when the screen of Fig. 19B is being displayed. On the Fig. 19C, a graphic representation 181 of soles that shows a load center with respect to the soles of the wearer is mainly provided, as shown at the center of Fig. 19C.

Further, an indicator 182a (RIGHT KNEE) of the joint name that is currently selected, a control mode indicator 182b (CVC), a sensitivity level indicator 182c (A1), and a torque limit 182d (30%) are displayed at an upper part relative to the graphic indicator 181 as the center.

Load amount indicators 183 (183a to d) are displayed at both edges on the left and right of the graphic representation 181 of the soles. The load amount indicators 183 (183a to d) show, in a bar graph format, which regions a load is applied to among the left, right, front and rear of the soles. Further, as operation phase indicators 184, displays ("SUPPORT" and "SWING") are provided that determine whether a leg is a supporting leg or a swinging leg based on the load on the soles. In addition, in the bottom row in an image 180, a torque tuner setting value 185 of the selected joint and a balance tuner setting value 186 of the selected joint are displayed. Furthermore, in a case where the task is walking, an indicator 187 showing the number of steps is provided on the right side of the bottom row in the image 180.

When an assistant performs settings of any drive portion among the drive portion 71L for the left hip joint, the drive portion 72L for the left knee joint, the drive portion 71R for the right hip joint and the drive portion 72R for the right knee joint on the screen 160, the screen 170 and the screen 190 during assistance as described above, first the assistant selects the relevant joint button among the joint buttons 143a to 143d corresponding to the respective drive portions on the operation unit 140. For example, when the joint button 143a is selected, as shown in Figs. 19A to 19C, indicators 161a, 175 and 192 of "R KNEE" are shown. In this state, it is possible to adjust the torque tuner setting value of the selected joint and the balance tuner setting value of the selected joint by pressing the upper, lower, left or right buttons of the arrow keys 146.

More specifically, pressing the upper and lower buttons of the arrow keys 146 adjusts the size of the torque tuner setting values 167d, 175, and 185 of the selected joint in Fig. 19A, Fig. 19B and Fig. 19C, respectively. The upper button of the arrow keys 146 is pressed to increase the torque tuner setting value, and the lower button of the arrow keys 146 is pressed to decrease the torque tuner setting value. Further, pressing the right and left buttons of the arrow keys 146 adjusts a flexion or an extension of the balance tuner setting values 168d, 176 and 186 of the selected joint in Fig. 19A, Fig. 19B and Fig. 19C, respectively. The left button of the arrow keys 146 is pressed to set the balance tuner setting value to the flexion side, and the right button of the arrow keys 146 is pressed to set the balance tuner setting value to the extension side.

### G. Control System

The control system includes the drive motors contained in the hip-joint drive portions 13L and 13R and the knee-joint drive portions 14L and 14R, joint angle sensors that detect a joint rotational angle, sole-pressure sensors that detect a sole pressure, a trunk absolute angle sensor that detects a trunk absolute angle, a biopotential signal sensor that detects a biopotential signal, and the control portion 18a that controls the drive motors in accordance with detection results of these sensors.

An angle sensor that detects a joint rotational angle is, for example, contained in each of the drive portions 13L, 13R, 14L and 14R. This angle sensor is constituted by, for example, a potentiometer that outputs a voltage that is proportional to a joint angle between a hip joint and a knee joint, and outputs a voltage signal in accordance with the joint rotational angle as a sensor output.

Specifically, an angle sensor contained in the hip-joint drive portion 13L detects a rotational angle between the hip frame 11 and the left thigh frame 22L that corresponds to a joint angle of the hip joint on the left side of the wearer. An angle sensor contained in the hip-joint drive portion 13R detects a rotational angle between the hip frame 11 and the right thigh frame 22R that corresponds to a joint angle of the hip joint on the right side of the wearer.

Further, an angle sensor contained in the knee-joint drive portion 14L detects a rotational angle between the lower end of the left thigh frame 22L and the left lower leg frame 23L that corresponds to a joint angle of the knee joint on the left side of the wearer. An angle sensor contained in the knee-joint drive portion 14R detects a rotational angle between the lower end of the right thigh frame 22R and the right lower leg frame 23R that corresponds to a joint angle of the knee joint on the right side of the wearer.

Sole-pressure sensors that detect a sole pressure are provided at a front part and a rear part on the back of the shoes 60L and 60R, and detect changes in loads applied to the back of the feet. The trunk absolute angle sensor is provided, for example, inside the hip frame 11, and detects changes in a trunk absolute angle of the wearer. The biopotential signal sensor has electrodes that are attached to respective parts of the hip joints and knee joints, and detects a biopotential signal that is generated between the electrodes.

The control portion 18a makes an overall determination of detection information of the angle sensors, sole-pressure sensors, trunk absolute angle sensor and biopotential signal sensor, and identifies an operation stage, that is, a phase, of a task that is selected by means of the task selection buttons 142 of the operation unit 140. For example, in the case of a cybernic voluntary control mode (CVC mode) that controls an assistance torque based on the strength of a biopotential signal, the control portion 18a controls the drive portions 13L, 13R, 14L and 14R so as to generate an assistive force (driving force) that is based on the identified phase, biopotential signals, the posture and other settings information. Note that, the size of the driving torque can be adjusted using the above described joint buttons 143 or arrow keys 146 or the like.

A configuration may also be adopted in which, in accordance with a setting operation performed using the operation unit 140, the control portion 18a determines the posture of the wearer by making an overall determination of detection information of the angle sensors, sole-pressure sensors, trunk absolute angle sensor and biopotential signal sensor, irrespective of the task that is selected by use of the task selection buttons 142, and switchably selects a task based on the determined result and controls the drive portions 13L, 13R, 14L and 14R so as to generate an assistive force in accordance with the relevant task.

Note that, the present invention is not limited to a case in which biopotential signals are used, and a configuration may also be adopted in which the control portion 18a detects a posture based on joint angles, sole pressures and trunk absolute angles, and controls the drive portions 13L, 13R, 14L and 14R based on the detected posture so as to generate an assistive force in accordance with pre-programmed leg trajectories. In addition, the control portion 18a may calculate assistive forces to be generated by the drive portions 13L, 13R, 14L and 14R based on only biopotential signals, without identifying a phase.

### H. Actions and Effects

In the action-assistance device 10, since the degree of opening (breadth) of the hip frame 11 constituting the lower limb frame 11 can be adjusted, the lower limb frame 11 can be fitted to and worn on the hips of various wearers of different body types and sizes.

Further, as shown in Fig. 2, in the action-assistance device 10, the shape of each portion of the lower limb frame 11 can be changed by bending the left thigh frame 22L, the right thigh frame 22R, the left lower leg frame 23L and the right lower leg frame 23R to the left or right (directions of arrows A, B, C, D and E), respectively, at the positions of the variable shape portions 41L, 41R, 42L, 42R, 43L, 43R, 44L, 44R, 45L and 45R.

In this embodiment, the action-assistance device 10 has the variable shape portions 41L, 41R, 42L, 42R, 43L, 43R, 44L, 44R, 45L and 45R at a total of 10 places which consist of five places on each of the right lower limb frame 21R and the left lower limb frame 21L, and can thus be fitted to and worn by various wearers who have respectively different leg shapes and sizes by appropriately changing the shape of any of the variable shape portions among the variable shape portions 41L, 41R, 42L, 42R, 43L, 43R, 44L, 44R, 45L and 45R so as to match the shape and size of the legs of the wearer. According to this configuration, the shape of each portion of the lower limb frame 11 can be changed to fit the leg shapes of, for example, even knock-kneed and bowlegged wearers.

Furthermore, in the action-assistance device 10, since the lengths of the left thigh frame 22L, the right thigh frame 22R, the left lower leg frame 23L and the right lower leg frame 23R can be respectively adjusted, the lower limb frame 11 can be fitted to and worn on lower limbs of various wearers who have different leg lengths.

Further, in the action-assistance device 10, since the left thigh frame 22L and the right thigh frame 22R are connected to the hip frame in a manner in which the left thigh frame 22L and the right thigh frame 22R are turnable around the respective long axes thereof via the hip portion connection mechanisms 25L and 25R, the action-assistance device 10 can assist actions of the wearer while allowing turning motions (inward turning and outward turning) of the hip joints of the wearer. Furthermore, because turning angles of the left thigh frame 22L and the right thigh frame 22R are limited to a range of a predetermined angle or less as the result of providing the turn limiting mechanism 30 in the action-assistance device 10, the hip joints of the wearer can be prevented from turning excessively, and actions of the wearer can be safely assisted.

In addition, in the action-assistance device 10, since the thigh cuffs 15 are provided on the left thigh frame 22L and the right thigh frame 22R, and the lower-leg cuffs 16 are provided on the left lower leg frame 23L and the right lower leg frame 23R, assistive forces generated by the drive portions 13L, 13R, 14L and 14R can be caused to act efficiently on the lower limbs of the wearer from the front or rear to assist the actions of the wearer.

Further, in the action-assistance device 10, since the front and back positions of the thigh cuffs 15 and the lower-leg cuffs 16 with respect to the lower limb frame 12 can be changed by inverting the front and rear sides of the thigh cuff support mechanisms 100 and the lower-leg cuff support mechanisms 200, respectively, the thigh cuffs 15 and the lower-leg cuffs 16 can be disposed at positions at which the thigh cuffs 15 and the lower-leg cuffs 16 can efficiently apply assistive forces to the lower limbs of the wearer, and thus actions of the wearer can be efficiently assisted.

Furthermore, in the action-assistance device 10, the height positions at which the thigh cuffs 15 and the lower-leg cuffs 16 are provided on the lower limb frame 12 can be adjusted by respectively adjusting attachment positions in the vertical direction of the thigh cuff support mechanisms 100 with respect to the thigh frame main body 71U and attachment positions in the vertical direction of the lower-leg cuff support mechanisms 200 with respect to the lower leg frame main body 72U, and hence the thigh cuffs 15 and the lower-leg cuffs 16 can be disposed at height position at which the thigh cuffs 15 and the lower-leg cuffs 16 can efficiently apply assistive forces to the lower limbs of the wearer, and actions of the wearer can thus be efficiently assisted.

In addition, in the action-assistance device 10, since angles of the thigh cuffs 15 and the lower-leg cuffs 16 with respect to the lower limb frame 12 can be adjusted by adjusting the angles of the arm members 133 of the thigh cuff support mechanisms 100 and the arm members 233 of the lower-leg cuff support mechanisms 200 with respect to the lower limb frame 12, respectively, the relevant angles can be adjusted to angles that can efficiently apply assistive forces to the lower limbs of the wearer, and thus actions of the wearer can be efficiently assisted.

Further, in the action-assistance device 10, since the thigh cuffs 15 and the lower-leg cuffs 16 can be retracted to positions at which the thigh cuffs 15 and the lower-leg cuffs 16 do not interfere with the wearer by rotating the arm member 133 of the respective thigh cuff support mechanisms 100 and the arm member 233 of the respective lower-leg cuff support mechanisms 200, the action-assistance device 10 can be put on a wearer and taken off from the wearer with ease.

As described in the foregoing, the action-assistance device 10 can be fitted to and worn by various wearers having different body types and sizes, and can also easily be put on and taken off from a wearer.

By putting the action-assistance device 10 on, for example, a wearer who has difficulty in walking independently, such as a patient undergoing walking motion rehabilitation, a walking action of the wearer can be assisted.

At such time, the action-assistance device 10 detects a joint angle sensor, sole pressures, a trunk absolute angle and biopotential signals, and operates to impart driving forces from the drive portions based on the detected signals.

When a wearer who is wearing the action-assistance device 10 performs a walking motion with his/her own intention, a driving torque that is in accordance with an action angle of a knee joint of the wearer is imparted as an assistive force from the action-assistance device 10 and, for example, it is possible for the wearer to walk with half the muscular strength that is required in the case of normal walking. Accordingly, the wearer can walk by means of the resultant force of the wearer's own muscular strength and the driving torque from the drive portions.

At such time, the action-assistance device 10 controls so that an assistive force (motor torque) imparted in accordance with movement of the center of gravity due to the walking motion reflects the intention of the wearer. Therefore, the drive portions of the action-assistance device 10 are controlled to assist actions in accordance with the intention of the wearer.

The action-assistance device 10 can also assist actions other than a walking action, such as an action when the wearer stands up from a state in which the wearer is sitting in a chair, or an action when the wearer sits down in a chair from a standing state. In addition, the action-assistance device 10 can perform power assistance in a case where the wearer walks up or down a staircase.

Further, in the action-assistance device 10, the operation unit 140 is provided in the vicinity of the hip frame, and the operation buttons (142 to 147) of the operation unit 140 are provided towards the back face side of the wearer. Therefore, while the wearer is walking, it is possible for an assistant to set an assistive force of the action-assistance device using the operation buttons (142 to 147) of the operation unit 140 while assisting the wearer from the rear side. In particular, when the hip frame 30 is viewed from the back face side, the layout of the operation buttons (142 to 147) corresponds to the positions of the drive portions to be set, and it is therefore possible for the assistant to perform setting operations for the drive portions by performing intuitive operations while assisting walking of the wearer and checking the walking circumstances of the wearer.

In general, while the wearer is walking, an assistant performs assistance while supporting the action-assistance device or the body of the wearer. According to the present embodiment, since the operation unit 140 is detachably provided in the vicinity of the hip frame 30, the assistant can, for example, use their right hand to operate the operation unit 140 that is fixed in the vicinity of the hip frame 30 while holding the hip frame 30 in their left hand to support the assistant, and in this way can both assist the wearer and perform a setting operation in a compatible manner.

In addition, on the operation unit 140 of the action-assistance device 10, the arrow keys 146 and the confirmation operation button 147 are arranged between the buttons for performing settings relating to an assistive force for joints on the left side and the buttons for performing settings relating to an assistive force for joints on the right side. The assistant can, without any confusion, select and operate an operation button with which the assistant wishes to perform a drive setting of an assistive force. In particular, because the operation buttons that correspond to the left-side joints are provided on the left of the operation unit 140 and the operation buttons that correspond to the right-side joints are provided on the right of the operation unit 140, and furthermore the arrow keys 146 and the confirmation operation button 147 are provided between the buttons on the left and right sides, erroneous operation of the left and right buttons does not occur, and selection and confirmation of setting contents can also be smoothly and simply performed using the arrow keys 146 and the confirmation operation button 147.

Further, since the start button 145 and the stop button 144 are provided separately from each other on the left and right sides across the arrow keys 146 and the confirmation operation button 147, an unintentional operation of the start button 145 or the stop button 144, that is, erroneous operation thereof, can be avoided.

By providing the display 141 which displays the settings status of the device or the assistance status of the device on the operation unit 140 of the action-assistance device 10, it is possible for the assistant to make settings that are suitable for the wearer while visually observing the settings status or the assistance status as well as the circumstances of the wearer.

Since the operation unit 140 has the task selection buttons 142 for accepting operations to select tasks, the assistant can switch a task at a suitable timing by performing a selection operation using the task selection buttons 142 while also assisting the wearer.

Since indicator portions (task indicators 153 and 171c) that display tasks selected by the control portion 18a are provided on the operation unit 140, it is possible for the assistant to appropriately assist the wearer while visually observing the assistance status in accordance with a task that is automatically switched to.

### I. Other Embodiments

Although in the above described embodiment the variable shape portions 41L, 41R, 42L and 42R of the left thigh frame 22L, the right thigh frame 22R, the left lower leg frame 23L and the right lower leg frame 23R are provided in the vicinity of the drive portions 13L, 13R, 14L and 14R, it is also possible to provide the variable shape portions 41L, 41R, 42L and 42R at positions that are away from the drive portions 13L, 13R, 14L and 14R, for example, at intermediate positions in the vertical direction of the thigh frame main body 71U and the lower leg frame main body 72U. Further, the variable shape portions may be provided both in the vicinity of the drive portions 13L, 13R, 14L and 14R and at intermediate positions in the vertical direction of the thigh frame main body 71U and the lower leg frame main body 72U.

Further, although the control system in the above described embodiment controls the drive portions 13L, 13R, 14L and 14R based on action angles of the joints of the wearer and a center of gravity position, the present invention is not limited thereto, and a configuration may also be adopted in which the control system controls the drive portions 13L, 13R, 14L and 14R based on signals that result from actions of the wearer, such as changes in biopotentials that are measured from the skin surface due to muscular activity of the operator. By determining the necessary assistive force (driving torque) based on biopotentials that are measured from the skin surface, the drive portions 13L, 13R, 14L and 14R can be controlled even in a case in which, depending on the muscular strength of the wearer, there is almost no change in the action angles of joints due to a noticeable decline in the muscular strength.

Further, a device that has only either one of the right lower limb frame 21R and the left lower limb frame 21L is also included in the action-assistance devices 10 of the present invention. In the case of the action-assistance device 10 having only the right lower limb frame 21R, the shoe 60L on the left side is connected to the back face unit 18 through a cable. Further, in the case of the action-assistance device 10 having only the left lower limb frame 21L, the shoe 60R on the right side is connected to the back face unit 18 through a cable. In a case where the shoe 60R or 60L on a side on which there is no right lower limb frame 21R or no left lower limb frame 21L is not used, naturally a corresponding cable is unnecessary.

### List of Reference Signs

- 10: action-assistance device,
- 11: hip frame,
- 12: lower limb frame,
- 13L, 13R: hip-joint drive portion,
- 14L, 14R: knee-joint drive portion,
- 15L, 15R: thigh cuff (assistive force exerting member),
- 16L, 16R: lower-leg cuff (assistive force exerting member),
- 18a: control portion,
- 140: operation unit,
- 141: display,
- 142: task selection button,
- 143: joint button,
- 143a: left hip joint button,
- 143b: left knee joint button,
- 143c: right hip joint button,
- 143d: right knee joint button,
- 144: stop button,
- 145: start button,
- 146: arrow keys,
- 147: confirmation operation button,
- 33: hip width adjustment mechanism,
- 21R: right lower limb frame,
- 21L: left lower limb frame,
- 22L: left thigh frame,
- 23L: left lower leg frame,
- 24L: left foot frame,
- 22R: right thigh frame,
- 23R: right lower leg frame,
- 24R: right foot frame,
- 25L: hip portion connection mechanism,
- 25R: hip portion connection mechanism,
- 30: turn limiting mechanism,
- 41L, 41R: variable shape portion,
- 14L, 14R: knee-joint drive portion,
- 42L, 42R: variable shape portion,
- 43L, 43R: variable shape portion,
- 44L, 44R: variable shape portion,
- 45L, 45R: variable shape portion,
- 50L, 50R: foot joint mechanism,
- 100: thigh cuff support mechanism,
- 200: lower-leg cuff support mechanism

## Claims

1. A wearable action-assistance device comprising:
a hip frame that is to be worn on hips of a wearer;
a lower limb frame that is to be worn on a lower limb of the wearer;
a plurality of drive portions provided on the lower limb frame in correspondence with joints of the wearer; and
a control portion that controls the drive portions based on a signal that is caused by an action of the wearer,
wherein the lower limb frame has a variable shape portion that can bend a portion other than the drive portions to left and right to adjust a flexion angle thereof.

2. The wearable action-assistance device according to claim 1, wherein the hip frame is a member which is open in front and can accept the hips of the wearer and surround the hips from a rear part to both side parts on the left and right thereof, and has a hip width adjustment mechanism for adjusting a hip width.

3. The wearable action-assistance device according to claim 1, wherein:
the lower limb frame comprises:
a right lower limb frame which is to be worn on a right lower limb of the wearer; and
a left lower limb frame which is to be worn on a left lower limb of the wearer,
the right lower limb frame comprises:
a right thigh frame which is positioned on a right side of a right thigh of the wearer; and
a right lower leg frame which is positioned on a right side of a right lower leg of the wearer,
the left lower limb frame comprises:
a left thigh frame which is positioned on a left side of a left thigh of the wearer; and
a left lower leg frame which is positioned on a left side of a left lower leg of the wearer, and
the variable shape portion is provided at an intermediate portion in a vertical direction of each of the right thigh frame, the left thigh frame, the right lower leg frame and the left lower leg frame, and also in an upper vicinity of each of the drive portions.

4. The wearable action-assistance device according to claim 1, wherein:
the lower limb frame comprises:
a right lower limb frame which is to be worn on a right lower limb of the wearer,
the right lower limb frame comprises:
a right thigh frame which is positioned on a right side of a right thigh of the wearer; and
a right lower leg frame which is positioned on a right side of a right lower leg of the wearer, and
the variable shape portion is provided at an intermediate portion in a vertical direction of each of the right thigh frame and the right lower leg frame, and also in an upper vicinity of each of the drive portions.

5. The wearable action-assistance device according to claim 1, wherein:
the lower limb frame comprises:
a left lower limb frame which is to be worn on a left lower limb of the wearer,
the left lower limb frame comprises:
a left thigh frame which is positioned on a left side of a left thigh of the wearer; and
a left lower leg frame which is positioned on a left side of a left lower leg of the wearer, and
the variable shape portion is provided at an intermediate portion in a vertical direction of each of the left thigh frame and the left lower leg frame, and also in an upper vicinity of each of the drive portions.

6. The wearable action-assistance device according to claim 3 or 4, wherein the right thigh frame is connected to the hip frame via an elastic body, the right thigh frame being turnable around each long axis.

7. The wearable action-assistance device according to claim 3 or 5, wherein the left thigh frame is connected to the hip frame via an elastic body, the left thigh frame being turnable around each long axis.

8. The wearable action-assistance device according to claim 6, comprising a turn limiting mechanism that limits a turning angle of the right thigh frame with respect to the hip frame to a predetermined angle or less.

9. The wearable action-assistance device according to claim 7, comprising a turn limiting mechanism that limits a turning angle of the left thigh frame with respect to the hip frame to a predetermined angle or less.

10. The wearable action-assistance device according to claim 1, comprising an assistive force action exerting member that is attached to the lower limb frame to cause a force of the drive portion to act on the wearer from the front or rear.

11. The wearable action-assistance device according to claim 10, wherein the assistive force exerting member is configured to be attachable in such a manner that an orientation thereof with respect to the wearer can be appropriately changed to a forward-facing orientation or a rearward-facing orientation.

12. The wearable action-assistance device according to claim 10 or 11, wherein a height of an attachment position of the assistive force exerting member with respect to the lower limb frame is adjustable.

13. The wearable action-assistance device according to claim 10 or 11, wherein an angle of the assistive force exerting member with respect to the lower limb frame is adjustable.

14. The wearable action-assistance device according to any one of claims 10 to 13, wherein the assistive force exerting member is supported by the lower limb frame in such a manner that the assistive force exerting member is retractable to a position at which the assistive force exerting member does not interfere with the wearer.

15. The wearable action-assistance device according to any one of claims 10 to 14, wherein the assistive force exerting member is a member having a mounting face which is curved in an arc shape that can be arranged so as to fit to a lower limb of the wearer.
